# EUROPEAN PATENT APPLICATION

(11) **EP 0 972 500 A1**
(43) Date of publication of application: **19.01.2000**
(21) Application number: 98500248.4
(22) Date of filing: 16.11.1998
(51) Int. Cl.: A61F 5/455

(54) **A device for feminine micturation**

(30) Priority: 13.07.1998 AR 1003390
(71) Applicant: Tort, José Pedro, 1004 Buenos Aires (AR)
(72) Inventor: Tort, José Pedro, 1004 Buenos Aires (AR)
(74) Representative: Urizar Anasagasti, José Antonio

(57) **Abstract**

The present invention relate a device which main purpose is to provide women means that allow them to urinate standing up. For such end, it consists of a small tube 17 cm long, said tube having a biased cut end, where it has a ring made of an antiallergenic material, all of which provide women something like the artificial prolongation of the urethra.

## Description

The present invention concerns a device for feminine micturition, and its purpose is to solve the problem that women have, whatever their age, that need to urinate in a place that is not their home, and have to do so in a bathroom that is not the one of their own house.

The needs in the practical order that lead the inventor to his inventive creation, are inherent of women that, due to different causes, leave their homes by different means of transport such as airplane, train, bus, etc. and need to urinate.

The problem invention solves is that, in view of such need, it prevents women from seating on toilets of other people's bathrooms which not always are clean and hygienic from the bacteriological point of view, that can spread venereal diseases, skin, etc. infections that can occur while seating on the seat of the toilet, and to prevent this, one can use the invented device that, conveniently placed, allows women to urinate standing up, without problems at all.

The present invention consists of a new industrial product that allows women to urinate standing up by means of a simple device represented by two figures enclosed in this specification, said device comprising a small tube (1) 17 cm long and 2,5 cm. diameter, approximately, said tube having a biased cut end (2) at approximately 30°, being rigid of flexible, straight or curved and it is provided of a ring (3) at the biased cut end, said ring is made of an antiallergenic material, said ring also being soft and smooth, this allowing to place it to the feminine urinary meatus without cuasing discomfort or irritaton, with no complications, thus allowing to urinate comfortably in a standing position, in the same way as men do when effecting identical physiologic function.

Said tube has as an object to provide women, only at the moment of urination, something like the artificial extension of the urethra having the length of only 4 cm, as from the neck of the bladder up to the urinary meatus, located in the vulva, and thus, with said extension a woman can urinate comfortably and securely as does a man when realizing the same act, possibility of which a woman lacks due to her natural conformation.

In cases of women that go out to the country (journeys, mountaineering, trekking,etc) the device shall give much comfortableness and safety when urinating, using the adequate clothes, that is skirt with buttons or trousers with fly.

Many women, when going out hold back the urine until they get back home so as not to use other bathrooms than their own. This practice may cause dilatation of the bladder and even cystitis, all of which is solved and is avoided using the device of the present invention.

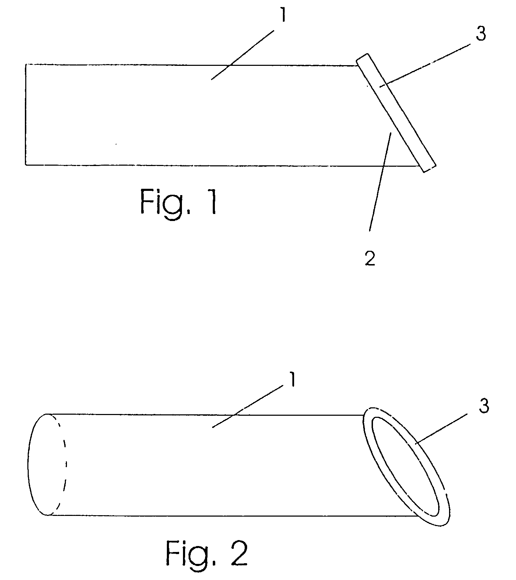

## Claims

1. Device for feminine micturition characterized because it includes a small tube, open at both ends, one of which is cut in biased manner and that has a ring adhered to the same.

2. Device for feminine micturition in accordance with claim 1 wherein it includes a ring made of antiallergenic material.

3. Device for feminine micturition in accordance with claim 1 wherein the ring is soft and smooth.
